(19)

Europäisches
Patentamt
European
Patent Office
Office européen
des brevets

(11) **EP 4 722 359 A1**

(12) **EUROPEAN PATENT APPLICATION**
published in accordance with Art. 153(4) EPC

(43) Date of publication:
08.04.2026 Bulletin 2026/15

(21) Application number: 24815613.5

(22) Date of filing: 31.05.2024

(51) International Patent Classification (IPC):
C12N 13/00 (2006.01)        C12M 1/00 (2006.01)
C12M 1/42 (2006.01)         C12N 15/09 (2006.01)
C12N 15/87 (2006.01)        C12P 1/00 (2006.01)

(52) Cooperative Patent Classification (CPC):
C12M 1/00; C12M 1/42; C12N 13/00; C12N 15/09;
C12N 15/87; C12P 1/00

(86) International application number:
PCT/JP2024/020041

(87) International publication number:
WO 2024/248133 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 01.06.2023  JP 2023090830
02.05.2024  JP 2024074810

(71) Applicant: FUJIFILM Corporation
Tokyo 106-8620 (JP)

(72) Inventors:
• KOBAYASHI, Yuka
Ashigarakami-gun, Kanagawa 258-8577 (JP)
• YAKUSHIJI, Takashi
Ashigarakami-gun, Kanagawa 258-8577 (JP)

(74) Representative: Hoffmann Eitle
Patent- und Rechtsanwälte PartmbB
Arabellastraße 30
81925 München (DE)

(54) **ELECTROPORATION METHOD, METHOD FOR PRODUCING USEFUL SUBSTANCE, FLOW PATH DEVICE, AND ELECTROPORATION APPARATUS**

(57)    An object is to provide an electroporation method capable of performing electroporation with high efficiency, a method for manufacturing a useful substance using the method, a flow channel device for performing the method, and an electroporation apparatus using the flow channel device. The object is achieved by, in the electroporation, forming sheath flows which flow together with a suspension and comes into contact with an electrode, and further having a restriction region which restricts a thickness of a suspension flow consisting of the suspension in a separation direction of an electrode pair to 1 to 10 mm.

## FIG. 1

EP 4 722 359 A1

**Description**

**BACKGROUND OF THE INVENTION**

1. Field of the Invention

The present invention relates to an electroporation method of introducing a bioactive substance into a biologically derived substance by applying an electric field to a suspension containing the biologically derived substance and the bioactive substance, a method for manufacturing a useful substance using the method, a flow channel device for performing the method, and an electroporation apparatus using the flow channel device.

2. Description of the Related Art

An electric perforating method is a method of introducing a substance into cells by perforating holes in a cell membrane with an electric pulse, and is also called electroporation. For example, fine holes can be made in the cell membrane by applying an electric field to a cell suspension using an electrode pair, and cells can be transformed by introducing deoxyribonucleic acid (DNA) into the cells.

A batch type is widely used as the electroporation for introducing a bioactive substance such as DNA, ribonucleic acid (RNA), and protein into a biologically derived substance such as a cell, a cell derivative, a cell organelle, an intracellular granule, and a vesicle by the electric perforating method.

In the batch type, for example, a suspension containing the biologically derived substance and the bioactive substance is accommodated in a container having an inner surface with the electrode pair installed thereon, and an electric field is applied by the electrode pair. As a result, fine holes are produced in the membrane covering a surface of the biologically derived substance, and permeability of the membrane is increased. In addition, the bioactive substance passes through the membrane with increased permeability by diffusion or electrophoresis, and thus the bioactive substance is introduced into the biologically derived substance.

On the other hand, as disclosed in JP2007-7430A and US11225638B, a flow type electroporation (flow electroporation) in which the suspension containing the biologically derived substance and the bioactive substance is circulated in a flow channel in which an electrode pair is installed has also been developed.

**SUMMARY OF THE INVENTION**

In the electroporation, as a concentration of the biologically derived substance such as cells in the suspension is higher, an utilization efficiency of the bioactive substance can be increased.

However, in the flow electroporation, in a case where the concentration of the biologically derived substance in the suspension is too high, an introduction efficiency may be decreased.

An object of the present invention is to provide an electroporation method capable of performing electroporation with high efficiency, a method for manufacturing a useful substance using the electroporation method, a flow channel device for performing the electroporation method, and an electroporation apparatus using the flow channel device.

In order to achieve the above-described object, the present invention has the following configurations.

[1] An electroporation method in which an electric field is applied to a suspension containing a bioactive substance and a biologically derived substance by an electrode pair to introduce the bioactive substance into the biologically derived substance, the electroporation method comprising:

forming sheath flows of a sheath liquid which flows together with the suspension and comes into contact with electrodes constituting the electrode pair, the sheath liquid being a liquid other than the suspension; and providing a restriction region through which only a suspension flow consisting of the suspension flows, in which a thickness of the suspension flow flowing through the restriction region in a separation direction of the electrodes constituting the electrode pair is 1 to 10 mm, and combining the suspension flow and the sheath flows downstream of the restriction region.

[2] The electroporation method according to [1],
in which a total thickness of the sheath flows in the separation direction of the electrodes in a case of combining with the suspension flow is equal to or less than the thickness of the suspension flow in the separation direction of the electrodes in the restriction region.

[3] The electroporation method according to [1],
in which, in a case where the sheath liquid is recovered downstream of the electrode pair, a dilution rate of the suspension in a mixed solution of the suspension and the sheath liquid after the sheath liquid is recovered is 2 times or less.

[4] The electroporation method according to any one of [1] to [3],
in which a volume fraction of the biologically derived substance in the suspension is 10% or more.

[5] The electroporation method according to [4],
in which the volume fraction of the biologically derived substance in the suspension is 40% or more.

[6] The electroporation method according to any one of [1] to [5],
in which the suspension is a cell suspension, and the sheath liquid is a culture medium used for culturing cells of the cell suspension.

[7] The electroporation method according to any one of [1] to [6],
in which, before the electric field is applied, a conductivity of the suspension or a concentration of the biologically derived substance in the suspension is measured, and the electric field or a pulse width applied to the electrode pair is adjusted according to a measurement result.

[8] The electroporation method according to any one of [1] to [7],
in which a flow rate of the suspension is 1 mL/min or more.

[9] The electroporation method according to any one of [1] to [8],
in which a flow rate of the sheath liquid is 0.1 mL/min or more.

[10] An electroporation method in which an electric field is applied to a suspension containing a bioactive substance and a biologically derived substance by an electrode pair to introduce the bioactive substance into the biologically derived substance, the electroporation method comprising:

forming sheath flows of a sheath liquid which flows together with the suspension and comes into contact with electrodes constituting the electrode pair, the sheath liquid being a liquid other than the suspension; and
setting a thickness of a suspension flow consisting of the suspension in a separation direction of the electrodes to 1 to 10 mm in at least a part in the electrode pair.

[11] A method for manufacturing a useful substance, comprising:
the electroporation method according to any one of [1] to [10].

[12] A flow channel device used for electroporation in which an electric field is applied to a suspension containing a bioactive substance and a biologically derived substance by an electrode pair to introduce the bioactive substance into the biologically derived substance, the flow channel device comprising:

a main flow channel;
an electrode pair which applies an electric field to a liquid flowing through the main flow channel;
a supply port which supplies the suspension to the main flow channel;
a suspension flow channel which has a restriction region through which only the suspension flows; and
a sheath liquid flow channel through which only a sheath liquid which is a liquid other than the suspension flows, in which the sheath liquid flow channel is disposed such that the sheath liquid flows downstream of the supply port and upstream of the electrode pair, and
a thickness of the restriction region in a separation direction of electrodes constituting the electrode pair is 1 to 10 mm.

[13] The flow channel device according to [12],
in which an angle formed by the main flow channel and the sheath liquid flow channel is 60° or less.

[14] The flow channel device according to [12] or [13],
in which the sheath liquid flow channel has a region in which the flow channel is widened in a width direction toward a downstream side.

[15] The flow channel device according to any one of [12] to [14],
in which a width of the main flow channel is 2 mm or more.

[16] The flow channel device according to any one of [12] to [15], further comprising, downstream of the electrodes:
a discharge port for discharging the sheath flows from the main flow channel.

[17] An electroporation apparatus comprising:

the flow channel device according to any one of [12] to [16]; and
a pair of contactors for energizing the electrodes of the flow channel device.

[18] A flow channel device used for electroporation in which an electric field is applied to a suspension containing a bioactive substance and a biologically derived substance by an electrode pair to introduce the bioactive substance into the biologically derived substance, the flow channel device comprising:

a main flow channel;

an electrode pair which applies an electric field to a liquid flowing through the main flow channel;

a supply port which supplies the suspension to the main flow channel; and

a sheath flow forming unit of forming sheath flows which come into contact with electrodes constituting the electrode pair downstream of the supply port in the main flow channel,

in which the sheath flow forming unit also functions as a restriction unit which restricts a thickness of a suspension flow flowing through the main flow channel in a separation direction of the electrodes constituting the electrode pair, and

a spacing of the restriction unit in the separation direction of the electrodes is 1 to 10 mm.

[19] The flow channel device according to [18],

in which the sheath flow forming unit has a sheath liquid flow channel which is provided upstream of the electrodes in the main flow channel and which is parallel to a flow direction of a liquid in the main flow channel, in order to supply a sheath liquid which forms the sheath flow, and

the sheath liquid flow channel acts as the restriction unit.

[20] The flow channel device according to [18] or [19],
in which a total thickness of the sheath liquid flow channel in the separation direction of the electrodes is equal to or less than a spacing of the sheath liquid flow channel in the separation direction of the electrodes.
[21] The flow channel device according to [18],

in which the sheath flow forming unit has a protrusion which protrudes in the separation direction of the electrodes in the main flow channel, and a sheath liquid supply port which supplies the sheath liquid to a downstream side in an oblique direction with respect to a flow direction of a liquid in the main flow channel, the sheath liquid supply port being opened downstream of the protrusion, and

the protrusion acts as the restriction unit.

[22] The flow channel device according to [21],
in which a total thickness of the protrusion in the separation direction of the electrodes is equal to or less than a spacing of the protrusion in the separation direction of the electrodes.
[23] The flow channel device according to any one of [18] to [22], further comprising, downstream of the electrodes:
a discharge port for discharging the sheath flows from the main flow channel.
[24] An electroporation apparatus comprising:

the flow channel device according to any one of [18] to [23]; and

a pair of contactors for energizing the electrodes of the flow channel device.

According to the present invention, electroporation can be performed with high efficiency.

## BRIEF DESCRIPTION OF THE DRAWINGS

FIG. 1 is a conceptual view for describing the electroporation method according to the embodiment of the present invention.

FIG. 2 is a schematic cross-sectional view of an example of the flow channel device according to the embodiment of the present invention.

FIG. 3 is a schematic top view of the flow channel device shown in FIG. 2.

FIG. 4 is a schematic cross-sectional view for describing an example of a flow channel substrate of the flow channel device shown in FIG. 2.

FIG. 5 is a conceptual view for describing the flow channel substrate shown in FIG. 4.

FIG. 6 is a conceptual view for describing an action of the flow channel device shown in FIG. 2.

FIG. 7 is a schematic cross-sectional view of another example of the flow channel device according to the embodiment of the present invention.

FIG. 8 is a conceptual view for describing an action of the flow channel device shown in FIG. 7.

FIG. 9 is a schematic cross-sectional view of still another example of the flow channel device according to the embodiment of the present invention.

FIG. 10 is a conceptual view for describing an example of feedback control in the electroporation method according to the embodiment of the present invention.

FIG. 11 is a conceptual view for describing an example of the feedback control in the electroporation method according to the embodiment of the present invention.

## DESCRIPTION OF THE PREFERRED EMBODIMENTS

Hereinafter, the electroporation method, the method for manufacturing a useful substance, the flow channel device, and the electroporation apparatus according to the present invention will be described in detail based on suitable examples shown in the accompanying drawings.

The drawings described below are exemplary for explaining the present invention, and the present invention is not limited to the drawings illustrated below.

In the following, "to" indicating the numerical range includes numerical values described on both sides.

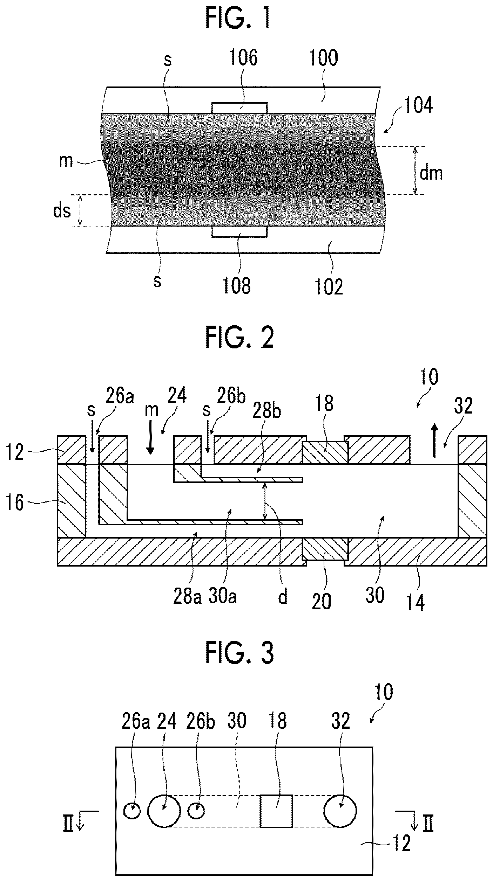

FIG. 1 conceptually shows the electroporation method according to the embodiment of the present invention.

In the present invention, a suspension flow consisting of a suspension m containing a biologically derived substance and a bioactive substance is caused to flow through a flow channel 104 provided between a first substrate 100 and a second substrate 102, and an electric field is applied to the suspension m by an electrode pair consisting of an electrode 106 and an electrode 108. Examples of the electric field include a pulsed electric field.

By applying the pulsed electric field, fine holes are formed in a membrane of the biologically derived substance, and the bioactive substance is introduced into the biologically derived substance by electrophoresis through the fine holes.

Here, in the present invention, as shown in FIG. 1, sheath flows consisting of a sheath liquid s different from the suspension are formed between the suspension flow and the electrodes 106 and 108 together with the suspension flow, and flow electroporation is performed.

By using such a sheath flow, even in a case where a concentration of the biologically derived substance in the suspension m is high, the flow of the biologically derived substance can be prevented from being retained in the vicinity of a wall surface of the flow channel 104, and thus the flow electroporation can be performed with high efficiency.

According to the studies of the present inventors, in a case where the concentration of the biologically derived substance in the suspension is high and the sheath flows are not present, the flow of the biologically derived substance is retained in the vicinity of the wall surface of the flow channel. In the retention layer, the biologically derived substance is adhered more densely than in a flowing region, and thus the electrical resistance is high. Therefore, the electric field applied by the electrode pair is largely consumed by the retention layer. As a result, the electric field applied to the flowing part, which should originally be applied with the electric field, is reduced, and a sufficient electric field cannot be applied, and thus an introduction efficiency of electroporation is reduced.

In addition, in a case where the sheath flows are not present, the biologically derived substance is retained as the retention layer in the vicinity of the electrodes, and thus the biologically derived substance is repeatedly applied with the electric field. As a result, the biologically derived substance is scorched in the vicinity of the electrodes, and the scorched biologically derived substance is attached to the surface of the electrodes. In a case where such foreign matter is attached to the surface of the electrodes, the electric field applied by the electrodes is shielded, and thus the introduction efficiency of electroporation is reduced.

Furthermore, as a result of the studies, the present inventors have found that, in the flow electroporation using such a sheath flow, high-efficiency flow electroporation can be performed by increasing a thickness dm of the suspension flow in a separation direction of the electrodes constituting the electrode pair.

Specifically, in the flow electroporation, by setting the thickness dm of the suspension flow in the separation direction of the electrodes constituting the electrode pair to 1 to 10 mm (1.0 to 10.0 mm; the same applies hereinafter), the high-efficiency flow electroporation can be performed. That is, in the example shown in the drawing, by setting the thickness dm of the suspension flow in the separation direction of the electrode 106 and the electrode 108 to 1 to 10 mm, the high-efficiency flow electroporation can be performed.

In the present invention, the thickness dm of the suspension flow may be 1 to 10 mm in a part between the electrode pair, or the thickness dm of the suspension flow may be 1 to 10 mm between the electrode pair and at an upstream end part of the electrodes. In addition, in the present invention, in terms of an area of one electrode exposed to the main flow channel, it is preferable that the thickness dm of the suspension flow is the above-described thickness in 30% or more of the region, it is more preferable that the thickness dm of the suspension flow is the above-described thickness in 50% or more of the region, and it is still more preferable that the thickness dm of the suspension flow is the above-described thickness in 70% or more of the region. In this case, since the two electrodes have the same size, either electrode may be used as a reference for one electrode in the electrode pair.

The electroporation method according to the embodiment of the present invention can perform the flow electroporation with high efficiency by increasing the thickness dm of the suspension flow in this way. Specifically, according to the present invention, the high-efficiency flow electroporation with high throughput, introduction efficiency, and utilization efficiency of the bioactive substance can be performed. The throughput is an amount of cells processed per unit time.

In addition, the electroporation method according to the embodiment of the present invention can perform large-scale flow electroporation efficiently on a high-concentration suspension while increasing the amount of processing by increasing the thickness dm of the suspension flow, thereby achieving high productivity.

In the electroporation method according to the embodiment of the present invention, a flow rate of the suspension (suspension flow) is not limited, but is preferably 1 mL/min or more, more preferably 2 mL/min or more, and still more preferably 3 mL/min or more. "mL" refers to milliliter.

It is preferable that the flow rate of the suspension is 1 mL/min or more from the viewpoint of increasing the amount of electroporation processing.

The flow rate of the suspension is preferably 1,000 mL/min or less.

In addition, in the electroporation method according to the embodiment of the present invention, A flow rate of the sheath liquid (sheath liquid flow) is not limited, but is preferably 0.1 mL/min or more. The flow rate of the sheath flow is a flow rate of the sheath flow on one electrode side. Therefore, the flow rate of the sheath flow is preferably 0.2 mL/min or more in total. The one electrode side is, in other words, one side of the electrode pair.

It is preferable that the flow rate of the sheath liquid is 0.1 mL/min or more from the viewpoint of suitably preventing the suspension from coming into contact with the electrodes.

It is preferable that the flow rate of the sheath flow is equal to or less than the flow rate of the suspension. It is preferable that the flow rate of the sheath flow is equal to or less than the flow rate of the suspension from the viewpoint of being able to suppress the amount of the sheath liquid, being able to suppress dilution of the suspension, and the like.

In the following description, the thickness of the suspension flow in the separation direction of the electrodes constituting the electrode pair is also simply referred to as "thickness of the suspension flow". The thickness of the sheath flow is also the same.

In addition, in the following description, the suspension having a high concentration of the biologically derived substance is also simply referred to as "high concentration suspension" or "high concentration of the suspension".

As described above, with the flow electroporation using the sheath flow, it is possible to perform an efficient treatment even in a case where the concentration of the suspension m is high. In the following description, the term "flow electroporation" refers to flow electroporation using a sheath flow unless otherwise specified.

Here, in the flow electroporation in the related art, the thickness of the suspension flow is made as thin as possible to perform the treatment.

For example, in US11225638B, as a preferred aspect, it is exemplified that the flow electroporation is performed by setting the thickness of the suspension flow, that is, the interval between the sheath flows to 50 to 100 $\mu$m. In US11225638B, it is disclosed that, as a result, the biologically derived substance (cell) passing between the electrodes can be made to flow in the flow channel as a single layer flow such that the biologically derived substance does not overlap in the separation direction of the electrodes.

In the flow electroporation in the related art, it is considered that the treatment with high efficiency can be performed by making the thickness of the suspension flow thin in this way, making the flow of the biologically derived substance between the electrodes uniform, and making the electric field applied to the biologically derived substance uniform.

However, as a result of the studies, the present inventors have found that, on the contrary, the flow electroporation with higher efficiency can be performed by making the thickness of the suspension flow significantly thicker.

In the flow electroporation, a diffusion region where the biologically derived substance and the bioactive substance are diffused is formed between the sheath flow and the suspension flow due to diffusion of the biologically derived substance and the bioactive substance contained in the suspension.

As in US11225638B, in a case where the thickness of the suspension flow is set to 50 to 100 $\mu$m, the diffusion region is thick with respect to the thickness of the suspension flow. As a result, the influence of the diffusion of the biologically derived substance and the bioactive substance on the flow electroporation is large, and thus the introduction efficiency and the utilization efficiency of the bioactive substance are reduced.

On the other hand, in a case where the thickness of the suspension flow is set to approximately 500 $\mu$m, the thickness of the diffusion region with respect to the thickness of the suspension flow is reduced, and the influence of the diffusion is reduced, but the uniformity of the electric field in the suspension flow is deteriorated. As a result, the introduction efficiency and the utilization efficiency of the bioactive substance are reduced as compared with the case in which the thickness of the suspension flow is set to 50 to 100 $\mu$m as in US11225638B.

On the other hand, by making the thickness dm of the suspension flow significantly thick as 1 mm or more, the influence of the diffusion of the biologically derived substance and the like can be suppressed.

That is, since the diffusion region is formed by the diffusion, the thickness of the diffusion region is not affected by the thickness dm of the suspension flow. Therefore, by setting the thickness dm of the suspension flow to 1 mm or more, the thickness of the suspension m, which is uniform and has no influence of the diffusion with respect to the thickness of the diffusion region, can be made sufficiently thick, and a large amount of the suspension m can be treated.

In addition, by setting the thickness dm of the suspension flow to 1 mm or more, electric field uniformity can also be improved. That is, in the electroporation, the strength of the electric field applied to the biologically derived substance fluctuates depending on the number of the biologically derived substances present in an inter-electrode direction. For example, as in US11225638B, in a case where the thickness of the suspension flow is sufficiently narrow as approximately 50 $\mu$m, the probability that the biologically derived substances overlap in the inter-electrode direction is low, and the electric field applied to each biologically derived substance is substantially constant, and the electric field uniformity is favorable. On the other hand, in a case where the thickness of the suspension flow is approximately 500 $\mu$m, the number of the biologically derived substances present in the inter-electrode direction varies depending on the position in the flow direction, and thus the electric field applied to each biologically derived substance varies, and the electric field uniformity is deteriorated. On the other hand, by making the thickness dm of the suspension flow sufficiently thick as 1 mm or more, the number of the biologically derived substances present in the inter-electrode direction at the position in the flow direction can be made uniform, and as a result, the electric field applied to each biologically derived substance can be made substantially constant, and the electric field uniformity can be improved.

As a result, with the electroporation method according to the embodiment of the present invention, it is possible to perform high-efficiency flow electroporation as compared with the flow electroporation in the related art, in which the suspension flow is made thin.

In the electroporation method according to the embodiment of the present invention, as in the flow channel device according to the embodiment of the present invention, which will be described later, a restriction region through which only the suspension flows is provided upstream of a position where the sheath flow and the suspension flow are combined in the flow channel 104, and a thickness of the restriction region, that is, a thickness of the suspension flow flowing through the restriction region is set to 1 to 10 mm. In other words, in the electroporation method according to the embodiment of the present invention, the suspension flow in which the thickness of the separation direction of the electrodes in the restriction region is set to 1 to 10 mm is formed, and the sheath flow is combined on both sides of the separation direction of the electrodes with the suspension flow in the downstream of the restriction region.

For example, in the flow channel device shown in FIG. 2 described later, a suspension flow channel 30a is the restriction region, and a spacing d in the separation direction of the electrodes in the region is set to 1 to 10 mm. It is sufficient that a part of the suspension flow channel 30a is the restriction region, and it is preferable that a part on the downstream side is the restriction region.

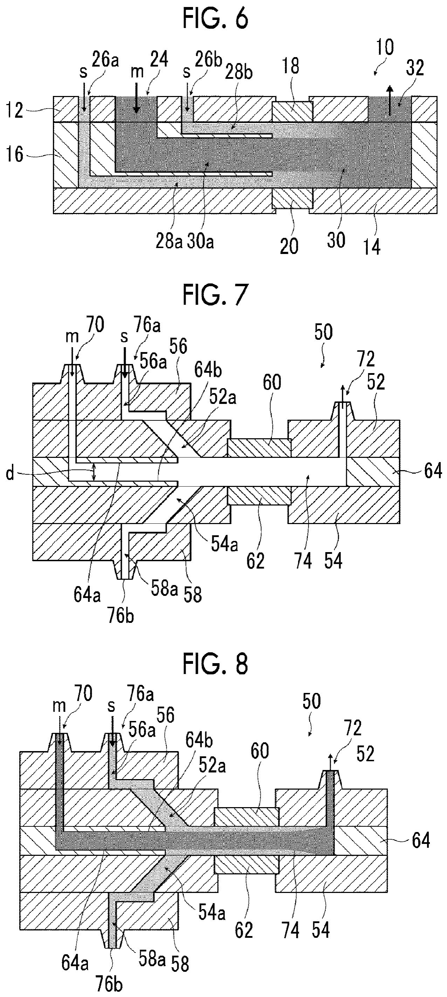

In addition, in a flow channel device 50 shown in FIG. 7 described later, a flow channel between a protrusion 64a and a protrusion 64b in a main flow channel 74 is the restriction region (hereinafter, this region is also referred to as a suspension flow channel), and a spacing d in the separation direction of the electrodes in the region is set to 1 to 10 mm. It is sufficient that a part of the suspension flow channel in FIG. 7 is the restriction region, and it is sufficient that a part on the downstream side is the restriction region and the spacing d is 1 to 10 mm.

The electroporation method according to the embodiment of the present invention can perform the high-efficiency flow electroporation by suitably setting the thickness dm of the suspension flow in the flow electroporation to 1 to 10 mm (approximately 1 mm to approximately 10 mm) by having such a configuration. It is preferable that the thickness dm of the suspension flow is set to 1 to 10 mm between the electrodes, that is, between the electrode 106 and the electrode 108, and thus the high-efficiency flow electroporation can be performed. In a case where the thickness dm of the suspension flow is sufficiently thick as 1 mm or more, the diffusion region between the sheath liquid and the suspension, generated between the electrodes, is extremely thin, although it depends on a distance from a supply port.

In the following description, the thickness of the restriction region in the separation direction of the electrodes is also simply referred to as "thickness of the restriction region".

In the electroporation method according to the embodiment of the present invention, in a case where the thickness of the restriction region is less than 1 mm, an inconvenience occurs in that the influence of the diffusion of the biologically derived substance and the like is large and an efficient treatment cannot be performed.

On the contrary, in a case where the thickness of the restriction region exceeds 10 mm, an inconvenience occurs in that the required applied voltage is high, a power supply device is large, heat generation is likely to occur (cooling efficiency is reduced), and a discharge phenomenon is likely to occur due to electric field concentration.

In the present invention, the thickness of the restriction region is preferably 1 to 8 mm, more preferably 2 to 5 mm, and still more preferably 2 to 3 mm. The lower limit of the thickness of the restriction region is more preferably 2 mm and still more preferably 3 mm.

In the present invention, it is preferable that the suspension flow and the sheath flow have a small difference in flow velocity in a case of combining the suspension flow and the sheath flow. Specifically, the flow velocity of the sheath flow in a case of combining the suspension flow and the sheath flow is preferably $\pm$10% or less, more preferably $\pm$5% or less, still more preferably $\pm$3% or less, and most preferably 0% (that is, the same speed) of the flow velocity of the suspension flow.

In addition, an angle between the suspension flow and the sheath flow in a case of combining the suspension flow and the sheath flow is preferably small. Specifically, the angle between the suspension flow and the sheath flow in a case of combining the suspension flow and the sheath flow is preferably 90° or less, more preferably 60° or less, still more preferably 45° or less, even more preferably 30° or less, and most preferably 0° (that is, parallel).

In the electroporation method according to the embodiment of the present invention, a thickness ds of the sheath flow by the sheath liquid s is not limited, but the thickness (one side) ds of the sheath flow in the separation direction of the electrodes constituting the electrode pair is set to 0.1 mm or more, whereby the high-efficiency flow electroporation can be performed. The thickness ds is preferably 0.2 mm or more, more preferably 0.3 mm or more, and particularly preferably 0.5 mm or more. The upper limit thereof is as follows.

Here, in the sheath flow, the total thickness in a case of combining with the suspension flow is preferably equal to or less than the thickness of the suspension flow in the restriction region. That is, the total thickness of the sheath flow in a case of combining with the suspension flow is preferably 1 time or less the thickness of the suspension flow in the restriction region.

In other words, a dilution rate of the suspension m downstream of the electrode pair is preferably 2 times or less.

In the flow channel device 10 shown in FIG. 2, it is preferable that the total thickness of a sheath liquid flow channel 26a and a sheath liquid flow channel 26b is equal to or less than a thickness of the suspension flow channel 30a. In addition, in the flow channel device 50 shown in FIG. 7, it is preferable that the total height of the protrusion 64a and the protrusion 64b is equal to or less than the gap d between the protrusion 64a and the protrusion 64b.

In the electroporation, in a case where an electric field is applied, holes are opened in the membrane (or shell) of the biologically derived substance at that time, and the bioactive substance enters the biologically derived substance by electrophoresis.

The holes formed in the membrane of the biologically derived substance gradually close over time, but in a case where, before the holes are closed, a liquid component (osmotic pressure, ion concentration, and the like) outside the biologically derived substance changes abruptly, for example, by an operation such as dilution of the suspension, the biologically derived substance is damaged by the movement of ions and the medium through the holes, and the ratio of components in the biologically derived substance changes abruptly, and the biologically derived substance is deformed and expanded.

As a result, for example, in a case of substance introduction into the cells, a survival rate of the biologically derived substance may decrease, and thus the introduction efficiency in electroporation and the utilization efficiency of the bioactive substance may decrease.

In a case where the thickness ds of the sheath flow is larger than the thickness dm of the suspension flow and the dilution rate of the suspension m is high, the survival rate of the biologically derived substance may decrease (damage may occur) and the EP efficiency may decrease, for example, in a case of the substance introduction into the cells. As a result of the studies by the present inventors, for example, in a case where a cell suspension is diluted with a culture medium sheath flow, it is usually observed that the cell survival rate is significantly decreased at a dilution rate of 2 times or more.

On the other hand, by setting the total thickness of the sheath flow in a case of combining with the suspension flow to be equal to or less than the thickness of the suspension flow in the restriction region, that is, setting the dilution rate of the suspension m to be 3 times or less, preferably 2 times or less, it is possible to perform an efficient treatment by suppressing the inconvenience caused by a large amount of the sheath liquid s being mixed into the biologically derived substance, for example, suppressing the decrease in the survival rate of the biologically derived substance in a case of the substance introduction into the cells.

From the viewpoint of more suitably obtaining the effect, the total thickness of the sheath flow in a case of combining with the suspension flow is more preferably 0.8 times or less and still more preferably 0.5 times or less the thickness of the suspension flow in the restriction region. That is, in the present invention, the dilution rate of the suspension m downstream of the electrode pair is more preferably 1.8 times or less and still more preferably 1.5 times or less.

The thickness (one side) of the sheath flow in a case of combining with the suspension flow is preferably 0.1 mm or more, more preferably 0.2 mm or more, still more preferably 0.3 mm or more, and particularly preferably 0.5 mm or more.

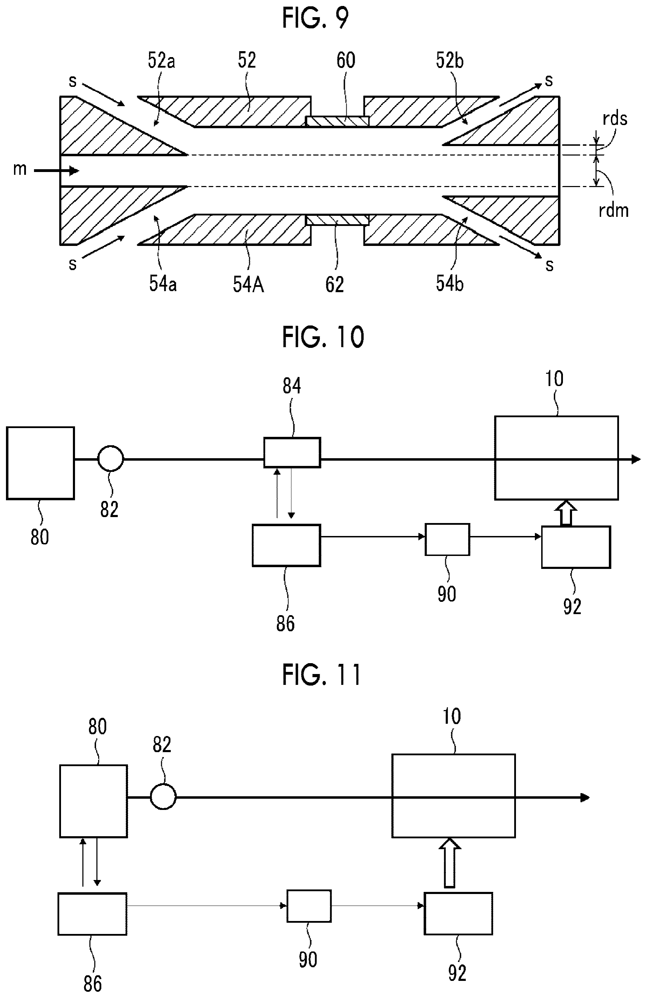

Alternatively, as shown in FIG. 9 described later, a discharge port for discharging the sheath liquid s (sheath flow) may be provided downstream of the electrode pair and the sheath liquid s may be recovered from the discharge port, so that the dilution rate of the suspension m downstream of the electrode pair may be set to 2 times or less. This configuration can also be used in the aspect of FIG. 1 described above.

According to the method, even in a case where the thickness of the sheath flow is larger than the thickness of the suspension flow, the dilution rate of the suspension treated by the electroporation method according to the embodiment of the present invention can be set to 2 times or less.

In the present invention, the method of setting the total thickness (ds × 2) of the sheath flow to be equal to or less than the thickness dm of the suspension flow and the method of recovering the sheath liquid s downstream of the electrode pair may be used in combination.

In the electroporation method according to the embodiment of the present invention, the concentration of the biologically derived substance in the suspension m is not limited.

Here, in the present invention, it is preferable that a volume fraction of the biologically derived substance in the suspension m is 10% or more.

In the electroporation, by increasing the concentration of the biologically derived substance in the suspension, the amount of the biologically derived substance to be treated can be increased as compared with a case in which the same amount of a low-concentration suspension is introduced. For example, in a case where the concentration is 2 times, the electroporation can be performed twice, and thus the throughput of the electroporation and the utilization efficiency of the biologically derived substance can be improved.

In addition, in the electroporation, as described above, the bioactive substance enters the biologically derived substance by electrophoresis. Therefore, even in a case where the concentration of the biologically derived substance is increased, the amount of the bioactive substance does not need to be increased in accordance with this, so that the utilization efficiency of the bioactive substance can also be improved.

In the present invention, by setting the volume fraction of the biologically derived substance in the suspension m to 10% or more, the above-described effects can be suitably obtained, and an efficient treatment can be performed.

The volume fraction of the biologically derived substance in the suspension m is more preferably 20% or more, still more preferably 30% or more, particularly preferably 40% or more, and most preferably 50% or more.

On the other hand, in the present invention, the volume fraction of the biologically derived substance in the suspension m is preferably 70% or less. In a case where the concentration of the suspension is too high, a viscosity of the cell suspension is rapidly increased, and the flow channel may be clogged. However, by setting the volume fraction of the biologically derived substance in the suspension m to 70% or less, the clogging of the flow channel due to the concentration of the suspension being too high can be suitably prevented.

The volume fraction of the biologically derived substance in the suspension m is more preferably 65% or less, and still more preferably 60% or less.

The electroporation method according to the embodiment of the present invention is basically the same as the known flow electroporation, except that the thickness of the suspension flow is 1 to 10 mm.

Therefore, the suspension, the sheath liquid, and various treatment conditions are not limited.

<Suspension>

In the present invention, the suspension is a suspension used for known electroporation, which contains a biologically derived substance, a bioactive substance, and a culture medium.

(Biologically derived substance)

The biologically derived substance is not particularly limited, and specific examples thereof include cells, cell organelles, intracellular granules and vesicles, and bacteria.

Among these, from the viewpoint that the effects of the present invention are more excellent, cells are preferable, animal cells are more preferable, mammalian cells are still more preferable, and cells derived from human or Chinese hamster are most preferable. Specific examples of the cells include human T cell, human embryonic kidney (HEK) 293 cell, A549, SF9, EB66, Daudi, Hela, Vero, MDCK, baby hamster kidney (BHK), Chinese hamster ovary (CHO), NS0, SP2/0, and hybridoma. In view of medicinal chemical manufacturing, gene introduction into HEK 293 cells or CHO cells is most commonly used.

(Bioactive substance)

The bioactive substance is a substance such as DNA, RNA, and protein, which exerts some action on a biologically derived substance by being introduced into the biologically derived substance.

Examples of the bioactive substance include plasmid DNA, linear DNA, mRNA, and protein. Among these, plasmid DNA, mRNA, linear DNA, or the like is preferred examples, and plasmid DNA is particularly preferable.

A concentration of the bioactive substance in the suspension is preferably 1 to 1,000 $\mu$g/mL, more preferably 2.5 to 500 $\mu$g/mL, and still more preferably 10 to 200 $\mu$g/mL.

The concentration of the bioactive substance in the suspension with respect to the culture medium in the suspension is preferably 10 to 500 $\mu$g/mL.

The concentration of the bioactive substance per biologically derived substance in the suspension is preferably 20 pg/cell or less, more preferably 5 pg/cell or less, still more preferably 1 pg/cell or less, and particularly preferably 0.5 pg/cell or less. As the concentration of the bioactive substance per biologically derived substance in the suspension is lower, the used amount of the bioactive substance per biologically derived substance can be reduced, so that the cost can be reduced.

<Sheath liquid>

As the sheath liquid used in the sheath flow, various liquids can be used as long as they are liquids other than the suspension.

Among these, the culture medium used in the suspension is preferably exemplified as the sheath liquid. The culture medium is preferably a culture medium used for proliferation of biologically derived substances such as cells.

<Voltage>

A voltage is set such that the electric field applied to the suspension is a desired electric field intensity, taking into account the thickness and the conductivity of the suspension flow, the thickness and the conductivity of the sheath flow, and the like, so that the electric field applied to the suspension between the electrodes is a desired electric field intensity.

The electrical resistance of the electrodes is preferably sufficiently lower than the resistance of the suspension and the sheath liquid because there is a possibility of causing a decrease in the introduction efficiency and the utilization efficiency of the bioactive substance in the electroporation.

An optimal value of the electric field applied to the suspension varies depending on the type and size of the biologically derived substance, but is usually approximately 100 to 3,500 V/cm, preferably approximately 500 to 3,000 V/cm, more preferably approximately 750 to 2,500 V/cm, and still more preferably approximately 1,000 to 2,000 V/cm.

The voltage is preferably a pulse voltage. In addition, the voltage may be a bipolar pulse in order to uniformize the electrode reaction. The electrode reaction is, for example, gas generation by electrolysis, electrode deterioration, and the like. In addition, the bipolar pulse is a pulse voltage in which positive and negative are alternately applied.

<Pulse width>

An optimum value of a pulse width varies depending on the type of the biologically derived substance, and the like, but is usually 0.1 to 100 ms (milliseconds), preferably approximately 1 to 10 ms.

<Pulse period (pulse interval)>

A pulse period is preferably synchronized with the time taken for the biologically derived substance to pass through the electrodes (electrode length L). For example, a pulse voltage is applied an average of 1 to 5 times, and preferably once while the biologically derived substance passes through the electrodes (electrode length L).

The time taken for the biologically derived substance to pass through the electrodes is determined by the flow rate and the flow channel cross-sectional area (flow channel width W of the main flow channel $\times$ electrode interval D). In addition, the pulse period is specifically preferably an integer multiple of the time taken for the biologically derived substance to pass through the electrodes.

The method for manufacturing a useful substance according to the embodiment of the present invention is a method including the electroporation method according to the embodiment of the present invention described above.

In the method for manufacturing a useful substance according to the embodiment of the present invention, the biologically derived substance is preferably cells, and the bioactive substance is preferably a plasmid DNA. As the plasmid DNA, plasmid DNA encoding a useful substance is used. By introducing the plasmid DNA into the cells by the electroporation method according to the embodiment of the present invention and culturing the cells into which the plasmid DNA has been introduced, the useful substance is produced in the cells.

The type of the useful substance in the present invention is not particularly limited, but is preferably a protein or a virus.

Examples of the useful substance include a recombinant polypeptide chain, a recombinant secreted polypeptide chain, an antigen-binding protein, a human antibody, a humanized antibody, a chimeric antibody, a mouse antibody, a bispecific antibody, an Fc fusion protein, a fragmented immunoglobulin, a single-chain antibody fragment (scFv), and a non-enveloped virus.

More specific examples of the non-enveloped virus include an adeno-associated virus, an adenovirus, a lentivirus, a baculovirus, and a retrovirus. The non-enveloped virus is known in the art and is described in WO2015/005430A, the contents of which are incorporated herein by reference.

The useful substance is preferably a human antibody, a humanized antibody, a chimeric antibody, a mouse antibody, or an adeno-associated virus.

The useful substance can be recovered and purified.

Regarding the recovery of the useful substance, the culture solution may be simply recovered; and for example, a liquid obtained by removing at least a part of the cells from the culture solution using a filter or a centrifuge may be recovered, and a known method is used without particular limitation. In a case where the purity of the useful substance is to be improved, the solvent is to be changed, or the form is to be changed, for example, to a powder, the culture solution or the above-described liquid can be subjected to further treatment.

In a case where the useful substance is a virus, virus particles produced in the cells are present in the culture solution or in the cells (in the nucleus). Extraction from the inside of the cells (destruction/dissolution of the cell membrane and the nucleic acid) may be performed by a freeze-drying method, or may be performed by adding a surfactant (such as Triton X) and stirring. Separation of the virus and the cell debris can be performed by centrifugation or depth filtration.

The useful substance can be purified by a purification treatment as necessary. The obtained useful substance can be purified to a high purity. For the separation and purification of the useful substance, a separation and purification method used for a normal protein or virus may be used.

For example, it is possible to carry out separation and purification of the useful substance by appropriately selecting and combining means such as a chromatography column such as affinity chromatography, a filter, ultrafiltration, salting out, dialysis, sodium dodecyl sulfate (SDS) polyacrylamide gel electrophoresis, electric focusing electrophoresis, and the like, which are not limited thereto.

In a case where the useful substance is a virus, the virus may be purified by performing TFF (concentration/buffer exchange), affinity chromatography, AEX (anion exchange chromatography) treatment, or the like as necessary. In this manner, it is possible to remove cell debris, cell-derived nucleic acids, and cell-derived proteins. Alternatively, a commercially available virus purification kit (such as an AAV purification kit) may be used. For example, an AAVpro (registered trademark) Purification Kit Maxi/Midi manufactured by Takara Bio Inc., or the like can be used.

The concentration of the useful substance obtained as described above can be measured according to absorbance measurement, enzyme-linked immunosorbent assay (ELISA), or the like.

In addition, in a case where the useful substance is an antibody, the titer of the antibody can also be measured with a commercially available analytical instrument such as Cedex Bio of F. Hoffmann-La Roche, Ltd.

In a case where the useful substance is a virus, the titer of the virus can be measured by a conventional method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding $MgCl_2$ and Benzonase to the collected supernatant to react. It is possible to measure the titer (vg/mL) of the virus by using the sample containing the target virus obtained as described above as a droplet digital PCR (ddPCR) sample, and performing ddPCR to measure the number of copies of intra-viral genome.

FIG. 2 conceptually shows a cross section of an example of the flow channel device according to the embodiment of the present invention, for performing the electroporation method according to the embodiment of the present invention.

A flow channel device 10 shown in FIG. 2 basically includes a first substrate 12, a second substrate 14, and a flow channel substrate 16 provided between the first substrate 12 and the second substrate 14.

An electrode 18 is incorporated into the first substrate 12 and an electrode 20 is incorporated into the second substrate to constitute an electrode pair.

The electrode 18 and the electrode 20 are composed of, for example, a metal material. The metal material is not particularly limited, and from the viewpoint of thermal conductivity, suitable examples thereof include Mo (molybdenum), Nb (niobium), W (tungsten), and Ti (titanium); and Mo or Nb is more preferable.

In addition, various materials can be used for each substrate as long as the substrate has sufficient resistance to the suspension and the sheath liquid and can ensure the required rigidity. Examples of a material for forming the substrate include a resin material such as polystyrene, a polyether ether ketone (PEEK) material, a fluorine-based resin including polytetrafluoroethylene, polydimethylsiloxane (PDMS), polycarbonate, and polypropylene.

FIG. 3 shows a top view of the flow channel device 10. The cross-sectional view of the flow channel device 10 shown in FIG. 2 is a cross section taken along a line II-II in FIG. 3.

The top view is a view of the flow channel device 10 in a laminating direction of the first substrate 12, the second substrate 14, and the flow channel substrate 16.

As shown in FIGS. 2 and 3, the flow channel device 10 includes a suspension inflow path 24 into which the suspension flows, a main flow channel 30, a discharge port 32 for discharging the treated suspension after the flow electroporation, and sheath liquid inflow paths 26a and 26b into which the sheath liquid flows.

The main flow channel 30 is formed between the first substrate 12 and the second substrate 14. In the flow channel device 10, the liquid flows from left to right in the drawing.

A width of the main flow channel is preferably 2 mm or more. In the present invention, the width of the main flow channel is a length (maximum value) of the main flow channel in a direction orthogonal to the separation direction of the electrodes. Regarding this point, the same applies to the aspect shown in FIG. 7 described later.

The sheath liquid inflow path 26a is connected to a sheath liquid flow channel 28a below in the drawing. The sheath liquid flow channel 28a is a flow channel parallel to the flow direction of the liquid in the main flow channel 30, and is opened upstream of the electrode 20, preferably directly upstream. The sheath liquid inflow path 26b is connected to a sheath liquid flow channel 28b above in the drawing. The sheath liquid flow channel 28b is a flow channel parallel to the flow direction of the liquid in the main flow channel 30, and is opened upstream of the electrode 20, preferably at the same position as the end part of the electrode 20 on the upstream side in the flow direction. Hereinafter, this position is also referred to as "directly upstream".

The suspension inflow path 24 is connected to a suspension flow channel 30a between the sheath liquid flow channel 28a and the sheath liquid flow channel 28b. That is, the suspension flow channel 30a is formed by the sheath liquid flow channel 28a and the sheath liquid flow channel 28b, and is opened upstream of the electrode pair, preferably directly upstream. The open end of the suspension flow channel 30a is a supply port in the flow channel device according to the embodiment of the present invention, which supplies the suspension to the main flow channel 30.

Therefore, the main flow channel 30 is formed of three flow channels of the sheath liquid flow channel 28a, the suspension flow channel 30a, and the sheath liquid flow channel 28b on the upstream side of the electrode pair (the electrode 18 and the electrode 20).

In such a flow channel device 10, the flow channel substrate 16 may be formed by various known methods.

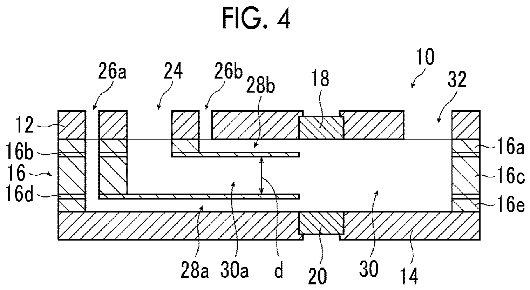

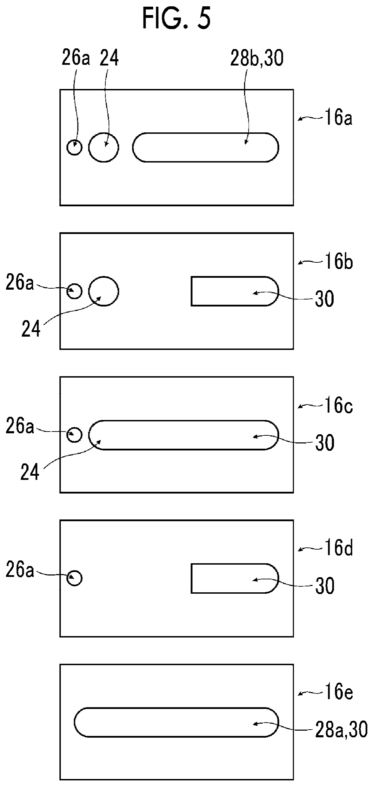

As an example, as shown in FIGS. 4 and 5, a method of forming the flow channel substrate 16 with five flow channel plates of a first flow channel plate 16a to a fifth flow channel plate 16e is exemplified.

The first flow channel plate 16a of the upper most layer in the drawing is a plate material having an opening corresponding to the sheath liquid inflow path 26a, an opening corresponding to the suspension inflow path 24, and an opening corresponding to the sheath liquid flow channel 28b and the main flow channel 30 on the upper side in the drawing.

The second flow channel plate 16b of the second layer from the top in the drawing is a plate material having an opening corresponding to the sheath liquid inflow path 26a, an opening corresponding to the suspension inflow path 24, and an opening corresponding to the main flow channel 30.

The third flow channel plate 16c of the third layer from the top in the drawing is a plate material having an opening corresponding to the sheath liquid inflow path 26a and an opening corresponding to the main flow channel 30.

The fourth flow channel plate 16d of the fourth layer from the top in the drawing is a plate material having an opening corresponding to the sheath liquid inflow path 26a and an opening corresponding to the main flow channel 30.

The fifth flow channel plate 16e of the fifth layer from the top in the drawing is a plate material having an opening corresponding to the sheath liquid inflow path 26a on the lower side in the drawing and an opening corresponding to the main flow channel 30.

In the flow channel device 10, the suspension m flows into the suspension inflow path 24, flows through the suspension flow channel 30a in the main flow channel 30, and reaches the discharge port 32 from the main flow channel 30.

On the other hand, the sheath liquid s flows into the sheath liquid flow channel 28a and the sheath liquid flow channel 28b. The sheath liquid s flowing into the sheath liquid flow channel 28a flows into the sheath liquid flow channel 28a on the lower side in the drawing in the main flow channel 30, and reaches the discharge port 32 from the main flow channel 30. On the other hand, the sheath liquid s flowing into the sheath liquid flow channel 28b flows into the sheath liquid flow channel 28b on the upper side in the drawing in the main flow channel 30, and reaches the discharge port 32 from the main flow channel 30.

Here, as described above, the sheath liquid flow channel 28a is a flow channel parallel to the flow direction of the liquid in the main flow channel 30, and is opened upstream of the electrode 20. In addition, the sheath liquid flow channel 28b is a flow channel parallel to the flow direction of the liquid in the main flow channel 30, and is opened upstream of the electrode 20.

Furthermore, the suspension flow channel 30a is formed by the sheath liquid flow channel 28a and the sheath liquid flow channel 28b, and is opened upstream of the electrode pair.

Therefore, as conceptually shown in FIG. 6, on the upstream side of the electrode pair, a sheath flow consisting of the sheath liquid s is formed on both sides of the suspension flow by the suspension m in the separation direction of the electrodes, that is, on the upper side and the lower side in the drawing, and the three-layer liquid flow of sheath flow/suspension flow/sheath flow is formed.

Therefore, by applying the pulsed electric field by the electrode 18 and the electrode 20 in a state of forming the sheath flow/suspension flow/sheath flow, the treatment by the flow electroporation using the sheath flow can be performed as described above.

Here, as described above, the suspension flow channel 30a is formed by the sheath liquid flow channel 28a and the sheath liquid flow channel 28b, and is opened upstream of the electrode pair, preferably directly upstream.

Therefore, the thickness of the suspension flow in the separation direction of the electrodes in the suspension flow channel 30a is determined by the spacing d between the sheath liquid flow channel 28a and the sheath liquid flow channel 28b. That is, the sheath liquid flow channel 28a and the sheath liquid flow channel 28b act as the restriction unit for the thickness of the suspension flow flowing through the suspension flow channel 30a.

Furthermore, the sheath liquid flow channel 28a and the sheath liquid flow channel 28b are flow channels parallel to the flow direction of the liquid in the main flow channel 30.

That is, in the flow channel device 10 of the example shown in the drawing, the suspension flow channel 30a is the restriction region which restricts the thickness of the suspension flow before the suspension flow is combined with the sheath flow.

Therefore, in the flow channel device 10, the thickness of the suspension flow in the separation direction of the electrodes, flowing through the suspension flow channel 30a, is set to 1 to 10 mm by setting the spacing d between the sheath liquid flow channel 28a and the sheath liquid flow channel 28b to 1 to 10 mm. Therefore, in the flow channel device 10, as described above, the thickness of the suspension flow can be controlled to 1 to 10 mm in the three-layer flow of the sheath flow/suspension flow/sheath flow in the flow electroporation.

As described above, the thickness of the suspension flow in the restriction region, that is, the spacing d is preferably 1 to 8 mm, more preferably 2 to 5 mm, and still more preferably 2 to 3 mm.

In addition, by setting the total thickness of the sheath liquid flow channel 28a and the sheath liquid flow channel 28b to be equal to or less than the spacing d between the sheath liquid flow channel 28a and the sheath liquid flow channel 28b, that is, the thickness of the suspension flow, the total thickness of the sheath flow can be set to be equal to or less than the thickness of the suspension flow, and the dilution rate of the suspension after the flow electroporation can be set to 2 or less.

FIG. 7 conceptually shows a cross section of another example of the flow channel device according to the embodiment of the present invention, for performing the electroporation method according to the embodiment of the present invention.

A flow channel device 50 shown in FIG. 7 basically includes a first substrate 52, a second substrate 54, a first introduction part 56, a second introduction part 58, and a flow channel substrate 64 provided between the first substrate 52 and the second substrate 54.

An electrode 60 is incorporated into the first substrate 52 and an electrode 62 is incorporated into the second substrate to constitute an electrode pair.

As shown in FIG. 7, the flow channel device 50 includes a suspension inflow path 70 into which the suspension flows, a main flow channel 74, a discharge port 72 for discharging the treated suspension after the flow electroporation, and sheath liquid inflow paths 76a and 76b into which the sheath liquid flows.

The main flow channel 74 is formed in a substantially linear shape between the first substrate 52 and the second substrate 54. In the flow channel device 50, the liquid also flows from left to right in the drawing.

The suspension inflow path 70 is formed to penetrate the first introduction part 56 and the first substrate 52 in the up-down direction in the drawing, and is opened to an uppermost upstream part of the main flow channel 74.

In the main flow channel 74 (flow channel substrate 64), protrusions 64a and 64b are provided to protrude in the separation direction of the electrodes in the electrode pair, that is, in the separation direction of the electrode 60 and the electrode 62, from the uppermost upstream part toward the downstream side.

The sheath liquid inflow path 76a is opened to the main flow channel 74 through a substantially L-shaped through-hole 56a provided in the first introduction part 56 and through a through-hole 52a provided in the first substrate 52. In the example shown in FIG. 7, the through-hole 56a and the through-hole 52a are the sheath liquid flow channel in the present invention.

Here, the through-hole 52a is provided to be inclined in a downstream direction with respect to the flow direction of the liquid in the main flow channel 74, and is opened to a position directly downstream of the protrusion 64a of the main flow channel 74. That is, the sheath liquid flow channel is provided to be inclined in the downstream direction with respect to the flow direction of the liquid in the main flow channel 74, and is opened to the downstream side of the supply port of the suspension and the upstream side of the electrode pair such that the sheath liquid flows in.

In the example shown in FIG. 7, a region where only the suspension flows on the upstream side of the main flow channel 74 is the suspension flow channel in the present invention; and a region sandwiched between the protrusion 64a and the protrusion 64b of the downstream part of the suspension flow channel is the restriction region in the present invention.

The through-hole 56a (a part of the sheath liquid flow channel) may be widened in a width direction from a position where the substantially L-shaped flow channel is bent toward the downstream side such that the sheath flow is uniform in the width direction. The same applies to the through-hole 58a described later. That is, it is preferable that the sheath liquid flow channel includes a region where the flow channel is widened in the width direction toward the downstream side. As a result, even in a case where the width of the main flow channel is wide, the flow velocity distribution is uniform in the downstream side, and the electroporation can be performed uniformly on the suspension. In this case, it is preferable that the suspension flow channel also includes a region where the flow channel is widened in the width direction toward the downstream side. As a result, the flow velocity distribution is also uniform in the suspension flow channel.

In addition, the through-hole 56a may be provided across the first introduction part 56 and the first substrate 52.

On the other hand, the sheath liquid inflow path 76b is opened to the main flow channel 74 through a substantially L-shaped through-hole 58a provided in the second introduction part 58 and through a through-hole 54a provided in the second substrate 54. Here, the through-hole 54a is provided to be inclined in a downstream direction with respect to the flow direction of the liquid in the main flow channel 74, and is opened to a position directly downstream of the protrusion 64b of the main flow channel 74. As with the through-hole 56a and the through-hole 52a, the through-hole 58a and the through-hole 54a are also the sheath liquid flow channel in the present invention. Therefore, the description of the through-hole 56a and the through-hole 52a also applies to the through-hole 58a and the through-hole 54a.

The through-hole 58a may be provided across the second introduction part 58 and the second substrate 54.

Therefore, the downstream side end part of the protrusion 64a and the protrusion 64b is the supply port in the flow channel device according to the embodiment of the present invention, which supplies the suspension to the main flow channel 74. In addition, the opening of the through-hole 52a to the main flow channel 74 and the opening of the through-hole 54a to the main flow channel 74 are the supply port of the sheath liquid to the main flow channel 74 in the flow channel device 50 according to the present invention. It is preferable that the widths of the supply port of the suspension and the supply port of the sheath liquid are substantially the same.

In such a flow channel device 50, the flow channel substrate 64 may be formed by various known methods.

In addition, as in the flow channel substrate 16, a plurality of flow channel plates may be used for forming the flow channel substrate 64. As an example, the flow channel substrate 64 may be formed of three flow channel plates, that is, a flow channel plate having a portion (non-opening portion) corresponding to the protrusion 64a and having an opening corresponding to the main flow channel 74, a flow channel plate having an opening corresponding to the entire main flow channel 74, and a flow channel plate having a portion (non-opening portion) corresponding to the protrusion 64b and having an opening corresponding to the main flow channel 74.

In the flow channel device 50, the suspension m flows into the suspension inflow path 70, flows from the most upstream part into the main flow channel 74, and reaches the discharge port 72 from the main flow channel 74.

On the other hand, the sheath liquid s flows into the sheath liquid inflow path 76a and the sheath liquid inflow path 76b. The sheath liquid s flowing into the sheath liquid inflow path 76a flows into the main flow channel 74 through the through-hole 56a provided in the first introduction part 56 and the through-hole 52a provided in the first substrate 52, and reaches the discharge port 72 from the main flow channel 74. In addition, the sheath liquid s flowing into the sheath liquid inflow path 76b flows into the main flow channel 74 through the through-hole 58a provided in the second introduction part 58 and the through-hole 54a provided in the second substrate 54, and reaches the discharge port 72 from the main flow channel 74.

Here, as described above, in the main flow channel 74 (flow channel substrate 64), the protrusions 64a and 64b are provided to protrude in the separation direction of the electrodes in the electrode pair, that is, in the separation direction of the electrode 60 and the electrode 62, from the uppermost upstream part toward the downstream side. That is, in the region having the protrusion 64a and the protrusion 64b, the thickness of the suspension m is the spacing between the protrusion 64a and the protrusion 64b, that is, the thickness d.

In addition, as described above, the through-hole 52a is provided to be inclined in a downstream direction with respect to the flow direction of the liquid in the main flow channel 74, and is opened to a position directly downstream of the protrusion 64a of the main flow channel 74. As described above, the region sandwiched by the protrusions is the restriction region in the present invention. On the other hand, the through-hole 54a is provided to be inclined in a downstream direction with respect to the flow direction of the liquid in the main flow channel 74, and is opened to a position directly downstream of the protrusion 64b of the main flow channel 74.

In FIG. 7, the sheath liquid flow channel is composed of the through-hole 56a (58a) and the through-hole 52a (54a), but only the through-hole 52a (54a) may be provided as the sheath flow channel, and the flow channel configuration may be appropriately designed according to the device configuration.

Therefore, as conceptually shown in FIG. 8, on the upstream side of the electrode pair, a sheath flow consisting of the sheath liquid s is formed on both sides of the suspension flow by the suspension m in the separation direction of the electrodes, that is, on the upper side and the lower side in the drawing, and the three-layer liquid flow of sheath flow/suspension flow/sheath flow is formed and the three-layer laminated flow flows between the electrode pair.

Therefore, by applying the pulsed electric field by the electrode 60 and the electrode 62 in a state of forming the sheath flow/suspension flow/sheath flow, the treatment by the flow electroporation using the sheath flow can be performed as described above. In this case, the thickness of the suspension flow is substantially equal to the thickness of the restriction region, and the thickness dm of the suspension flow is maintained at 1 to 10 mm at least between the electrode pair and at the upstream end part of the electrode pair.

As described above, the sheath liquid s from the sheath liquid inflow path 76a flows into the directly downstream side of the protrusion 64a of the main flow channel 74 to be inclined toward the downstream side. On the other hand, the sheath liquid s from the sheath liquid inflow path 76b flows into the directly downstream side of the protrusion 64b of the main flow channel 74 to be inclined toward the downstream side.

In addition, on the upstream side of the inflow portion of the sheath liquid s, the protrusion 64a and the protrusion 64b are formed in the main flow channel 74, and the thickness of the main flow channel 74 in the separation direction of the electrodes is thinner than that on the downstream side of the inflow position of the sheath liquid s. In addition, the thickness of the main flow channel 74 in the separation direction of the electrodes, that is, the thickness of the suspension flow in the region is determined by the spacing between the protrusion 64a and the protrusion 64b.

Therefore, the protrusion 64a and the protrusion 64b constitute a sheath flow forming unit, and also act as the restriction unit which restricts the thickness of the suspension flow.

That is, in the flow channel device 50 of the example shown in the drawing, the region where the protrusion 64a and the protrusion 64b are formed in the main flow channel 74 is the restriction region which restricts the thickness of the suspension flow before the suspension flow is combined with the sheath flow.

Therefore, in the flow channel device 70, the thickness of the suspension flow flowing through the restriction region is set to 1 to 10 mm by setting the spacing d between the protrusion 64a and the protrusion 64b to 1 to 10 mm. Therefore, in the flow channel device 70, as described above, the thickness of the suspension flow can be controlled to 1 to 10 mm in the three-layer flow of the sheath flow/suspension flow/sheath flow in the flow electroporation.

As described above, the thickness of the suspension flow in the restriction region, that is, the spacing d is preferably 1 to 8 mm, more preferably 2 to 5 mm, and still more preferably 2 to 3 mm. The spacing d may be 2 to 10 mm or 3 to 10 mm.

In addition, by setting the total thickness of the protrusion 64a and the protrusion 64b, that is, the total height in the separation direction of the electrodes to be equal to or less than the spacing d between the protrusion 64a and the protrusion 64b, the total thickness of the sheath flow can be set to be equal to or less than the thickness of the suspension flow, and the dilution rate of the suspension after the flow electroporation can be set to be 2 times or less.

In the flow channel device 50, an angle formed by the suspension flow and the sheath flow in a case of combining the suspension flow and the sheath flow, that is, an angle between the main flow channel 74 and the through-hole 52a and the through-hole 54a is not limited, but is preferably small.

Specifically, as described above, the angle between the main flow channel 74 and the through-hole 52a and the through-hole 54a is preferably 90° or less, more preferably 60° or less, still more preferably 45° or less, and particularly preferably 30° or less.

As described above, in the present invention, the discharge port for discharging the sheath liquid s (sheath flow) may be provided downstream of the electrode pair and the sheath liquid s may be recovered from the discharge port, so that the dilution rate of the suspension m downstream of the electrode pair may be set to 2 times or less.

As an example, as conceptually shown in FIG. 9 in a manner similar to the flow channel device shown in FIG. 7, in the first substrate 52, a recovery path 52b which discharges the sheath liquid s from the sheath flow is provided downstream of the electrode 60. On the other hand, in the second substrate 54, a recovery path 54b which discharges the sheath liquid s from the sheath flow is provided downstream of the electrode 62.

In this way, the sheath liquid s can be recovered from the sheath flow downstream of the electrode pair, and the total thickness of the flow channels of the upper and lower sheath liquid flows after the recovery of the sheath liquid s, that is, the total thickness rds can be set to be thinner than the thickness rdm of the flow channel of the suspension flow. As a result, the dilution rate of the suspension treated by the flow electroporation can be set to 2 times or less. The thickness of the sheath liquid flow after the recovery may be controlled by, for example, the thickness of the recovery path.

In addition, according to the method, even in a case where the thickness of the sheath flow at the time of the combination is larger than the thickness of the suspension flow between the restriction unit, that is, in the restriction region, the dilution rate of the suspension after the flow electroporation can be set to 2 times or less.

As described above, in the electroporation method according to the embodiment of the present invention, for example, the flow electroporation is performed by applying the pulsed electric field to the suspension.

Here, in order to perform the appropriate flow electroporation, it is necessary to apply the appropriate pulsed electric field having an appropriate voltage at an appropriate pulse width (length of one pulse) according to the concentration of the suspension. The conditions for applying the electric field are determined in advance to be optimal according to the concentration of the suspension, the flow rate, the frequency of the set pulsed electric field, and the like.

However, the concentration of the suspension may also fluctuate due to a reason such as a difference in cell proliferation, a desired cell concentration cannot be obtained, or a desired cell concentration cannot be obtained due to a fluctuation in a case where continuous cell concentration or mixing of the bioactive substance is performed immediately before the electroporation.

In a case where the fluctuation of the suspension or the like occurs, the optimal conditions are different, and thus the appropriate flow electroporation cannot be performed even in a case where the pulsed electric field is applied under the predetermined conditions.

In response to this, in the present invention, it is preferable to perform feedback control of measuring the conductivity of the suspension to be supplied to the flow channel device and adjusting the electric field applied by the electrode pair according to the measurement result.

FIG. 10 conceptually shows an example thereof.

In FIG. 10, for example, the flow channel device 10 is used. In the example shown in FIG. 10, the suspension is supplied from a suspension container 80 to the flow channel device 10 (suspension inflow path 24) by a liquid feeding pump 82, and the flow electroporation is performed as described above.

Here, a measurement jig 84 is provided in a flow path of the suspension from the suspension container 80 to the flow channel device 10.

The measurement jig 84 measures, for example, a current waveform, a voltage, and the like of the passing suspension, and transmits the result to a conductivity calculation unit 86. The conductivity calculation unit 86 calculates the conductivity σm' of the suspension from the supplied current waveform or voltage, and sends the calculated conductivity σm' to an operation and power supply control device 90. The measurement jig 84 and the conductivity calculation unit 86 constitute a flow cell-type conductivity measurement device.

The operation and power supply control device 90 first calculates a correction value Ton' of a pulse width of the voltage to be applied or a correction value V0' of the voltage to be applied by using the supplied conductivity σm, and drives a pulse power supply 92 according to the correction value.

Specifically,

a pulse width set as a steady state condition is denoted as Ton,
a voltage set as a steady state condition is denoted as V0,
a conductivity of the assumed suspension is denoted as σm,
a conductivity of the assumed sheath liquid is denoted as σs,
a thickness of the assumed suspension flow is denoted as dm, and
a thickness of the assumed sheath flow is denoted as ds.

On this basis, the operation and power supply control device 90 calculates the correction value Ton' of the pulse width of the pulsed electric field to be applied by the following expression from the measured conductivity σm' of the suspension.

$$\text{Ton'} = \text{Ton} \cdot (\sigma m' \cdot 2ds + \sigma s \cdot dm)/(\sigma m \cdot 2ds + \sigma s \cdot dm)$$

Alternatively, the operation and power supply control device 90 calculates the correction value V0' of the voltage of the pulsed electric field to be applied by the following expression from the measured conductivity σm' of the suspension.

$$\text{V0'} = \text{V0} \cdot (\sigma m' \cdot 2ds + \sigma s \cdot dm)/(\sigma m \cdot 2ds + \sigma s \cdot dm)$$

Next, the operation and power supply control device 90 drives the pulse power supply 92 according to the calculated correction value Ton' of the pulse width or the calculated correction value V0' of the voltage.

The pulse power supply 92 supplies the pulse potential having a pulse width corresponding to the supplied correction value or the pulse potential having a voltage corresponding to the supplied correction value, to the electrode pair (the electrode 18 and the electrode 20) to perform the flow electroporation.

In the present invention, by performing such feedback control, it is possible to perform appropriate flow electroporation even in a case where the concentration of the suspension fluctuates.

In the example shown in FIG. 10, the feedback control is performed online, but the present invention is not limited thereto, and the feedback control may be performed offline. FIG. 11 shows an example thereof.

In the example shown in FIG. 11, the conductivity σm' of the suspension to be filled in the suspension container 80 is measured by the conductivity calculation unit 86, instead of measuring the conductivity of the suspension in the flow channel from the suspension container 80 to the flow channel device 10 by the flow cell-type conductivity measurement device.

The measurement value is sent to the operation and power supply control device 90 as in the above, the correction value Ton' of the pulse width or the correction value V0' of the voltage is calculated, and the pulse power supply 92 is driven according to the correction value to perform the feedback control.

In addition, the feedback control may be performed by measuring the concentration of the suspension instead of the conductivity σm' of the suspension.

That is, a suspension concentration measurement device is provided instead of the flow cell-type conductivity measurement device in FIG. 10 and the conductivity calculation unit in FIG. 11, and the measurement result of the concentration of the suspension is sent to the operation and power supply control device 90.

The operation and power supply control device 90 uses a reference table or a logical expression indicating a relationship between the cell concentration and the conductivity to ascertain the conductivity σm' of the suspension from the cell concentration of the suspension.

After that, the correction value Ton' of the pulse width or the correction value V0' of the voltage is calculated using the calculated conductivity σm' of the suspension, and the pulse power supply 92 is driven according to the correction value to perform the feedback control.

The electroporation apparatus according to the embodiment of the present invention is an apparatus including the flow channel device according to the embodiment of the present invention, which performs the electroporation method according to the embodiment of the present invention, and a pair of contactors for energizing the electrodes of the flow channel device.

The electrodes included in the flow channel device according to the embodiment of the present invention do not need to have the entire surface exposed to the flow channel of the liquid (suspension (sheath liquid)), and may have a flow channel exposed part which is exposed to the flow channel and is in contact with the liquid flow and a non-exposed part which is not exposed to the flow channel.

In this case, it is preferable that the non-exposed part is a contact part which comes into contact with the contactors to energize the electrodes. For example, the electrode may be formed in an L-shape, and one end part of the L-shape may be the flow channel exposed part and the other end part may be the contact part.

As described above, the electroporation apparatus including the flow channel device according to the embodiment of the present invention includes a pair of contactors for energizing the electrodes included in the flow channel device.

In the flow channel device according to the embodiment of the present invention, for example, the flow channel of the liquid is formed by sandwiching a plate material for forming a flow channel, which has a groove serving as the flow channel, with a plate material for holding the electrode. The plate material for holding the electrode holds the electrode such that a part of the electrode is the flow channel exposed part and a part of the electrode is the contact part (non-exposed part). The plate material for holding the electrode has an opening for coming into contact with the contactor for energization at a position corresponding to the contact part.

In a case where the pair of contactors and the electrodes are brought into contact with each other, for example, one electrode of the pair of electrodes included in the flow channel device and one of the pair of contactors are substantially fixed in a state of being in contact with each other. Next, the other contactor fixed to an actuator is brought close to and comes into contact with the contact part of the other electrode of the flow channel device. Thereafter, the other contactor is fixed by a fixing unit, and the pair of electrodes and the pair of contactors are brought into contact with each other, and the flow channel device is set in the flow electroporation apparatus.

Although the electroporation method, the method for manufacturing a useful substance, the flow channel device, and the flow electroporation apparatus according to the embodiments of the present invention have been described in detail above, the present invention is not limited to the above-described embodiments, and various improvements or modifications may be made without departing from the spirit of the present invention.

Examples

Hereinafter, the characteristics of the present invention will be described in detail by Examples. The materials, reagents, amounts and proportions of substances, operations, and the like described in Examples can be appropriately modified as long as the gist of the present invention is maintained. Therefore, the scope of the present invention is not limited to Examples below.

[Example 1]

<Preparation of suspension>

A cell suspension (cell concentration: 40 Mcells/mL) of HEK293 cells (corresponding to a biologically derived substance) having an average diameter of 20 $\mu$m, which had been proliferated and cultured, was prepared. "mL" refers to milliliter as described above. The average diameter of the cells and the cell concentration were measured using Vi-CELL XR (Beckman Coulter, Inc.).

A pmaxGFP plasmid (corresponding to a bioactive substance) manufactured by Lonza was added to the obtained cell suspension so that a concentration of 30 $\mu$g/mL was reached. In this way, a suspension containing the HEK293 cells, the GFP plasmid, and the culture medium was obtained.

The cell volume fraction in the suspension was 17%, and the plasmid concentration was 30 $\mu$g/mL.

Assuming that the cells were spherical, the cell volume fraction in the suspension (= $4 \div 3 \times (20 \mu m \div 2)^3 \times \pi \times$ 40 Mcells/mL $\div 10^6 \times 100$ (%)) was calculated from the cell concentration (40 Mcells/mL) and the average diameter of the cells (20 $\mu$m).

Flow electroporation was performed using the flow channel device as shown in FIG. 2. As the sheath liquid, the culture solution of the suspension was used.

A thickness of the suspension flow was set to 2 mm, a thickness of the sheath flow was set to 2 mm (one side), and a flow channel width of the main flow channel was set to 2 mm. Therefore, in the present example, a dilution rate of the suspension was 3 times. The flow channel width of the main flow channel was a length in a separation direction of the electrodes, that is, a direction orthogonal to the thickness of the suspension flow or the like.

A flow rate of the suspension was set to 4 mL/min, and a flow rate (one side) of the sheath liquid was set to 4 mL/min.

In the present example, the thickness of the suspension flow was a thickness of the suspension flow in the suspension flow channel of the flow channel device, and the thickness of the sheath flow was a thickness of the sheath flow in the sheath liquid flow channel of the flow channel device. Regarding this point, the same applies to other examples.

[Example 2]

Flow electroporation was performed in the same manner as in Example 1, except that the thickness of the sheath flow was set to 0.5 mm (one side) and the pulse voltage was set to 381 mV. The flow rate (one side) of the sheath liquid was 1 mL/min.

Therefore, in the present example, a dilution rate of the suspension was 1.5 times.

[Example 3]

Flow electroporation was performed in the same manner as in Example 1, except that a recovery path was provided downstream of the electrode pair, and the sheath liquid was recovered from the sheath flow after passing through the electrode pair (see FIG. 9).

A thickness (one side) of the sheath liquid recovery path was 1.5 mm. That is, the thickness of the sheath flow after the sheath liquid recovery was 0.5 mm.

Therefore, in the present example, a dilution rate of the suspension was 1.5 times.

[Example 4]

Flow electroporation was performed in the same manner as in Example 2, except that the cell concentration in the suspension was set to 120 Mcells/mL, the cell volume fraction in the suspension was set to 50%, and the pulse voltage was set to 330 mV.

[Example 5]

Flow electroporation was performed in the same manner as in Example 2, except that the cell concentration in the suspension was set to 140 Mcells/mL, the cell volume fraction in the suspension was set to 59%, and the pulse voltage was set to 330 mV.

[Example 6]

Flow electroporation was performed in the same manner as in Example 5, except that, as shown in FIG. 10, the conductivity of the suspension was measured during the feeding of the suspension to the flow channel device, the pulse width of the pulsed electric field to be applied was feedback-controlled according to the measurement result, and the pulse voltage was set to 319 mV.

[Example 7]

Flow electroporation was performed in the same manner as in Example 5, except that, as shown in FIG. 10, the conductivity of the suspension was measured during the feeding of the suspension to the flow channel device, the pulse width of the pulsed electric field to be applied was feedback-controlled according to the measurement result, and the pulse width was set to 3.39.

[Comparative Example 1]

Flow electroporation was performed in the same manner as in Example 1, except that the thickness of the suspension flow was set to 0.1 mm, the thickness of the sheath flow was set to 0.1 mm (one side), the flow channel width of the main flow channel was set to 0.2 mm, the flow rate of the suspension was set to 0.02 mL/min, the flow rate of the sheath liquid (one side) was set to 0.2 mL/min, and the pulse voltage was set to 35 mV. Therefore, in the present example, a dilution rate of the suspension was 3 times.

[Comparative Example 2]

Flow electroporation was performed in the same manner as in Example 1, except that the thickness of the suspension flow was set to 0.5 mm, the thickness of the sheath flow was set to 0.1 mm (one side), the flow rate of the suspension was set to 0.02 mL/min, the flow rate of the sheath liquid (one side) was set to 0.2 mL/min, and the pulse voltage was set to 90 mV.

Therefore, in the present example, a dilution rate of the suspension was 1.4 times.

[Comparative Example 3]

Flow electroporation was performed in the same manner as in Example 1, except that the sheath flow was not formed, the cell concentration in the suspension was set to 120 Mcells/mL, the cell volume fraction in the suspension was set to 50%, and the pulse voltage was set to 275 mV.

In the present example, it was found that the cells (biologically derived substances) of the suspension were retained near the wall surface of the main flow channel.

[Comparative Example 4]

Flow electroporation was performed in the same manner as in Example 1, except that the thickness of the suspension flow was set to 0.1 mm, the thickness of the sheath flow was set to 0.1 mm (one side), the flow channel width of the main flow channel was set to 0.2 mm, the flow rate of the suspension was set to 0.02 mL/min, the flow rate of the sheath liquid (one side) was set to 0.2 mL/min, the cell concentration in the suspension was set to 120 Mcells/mL, the cell volume fraction in the suspension was set to 50%, and the pulse voltage was set to 24.7 mV.

Therefore, in the present example, a dilution rate of the suspension was 3 times.

In the present example, the main flow channel was blocked during the flow electroporation treatment, and the treatment could not be performed until the end.

The above examples are summarized in Table 1 below.

In Table 1, "Plasmid concentration (in culture medium)" indicates the concentration of the GFP plasmid in the suspension with respect to the culture medium in the suspension (calculated value).

In addition, "Plasmid concentration per cell" in Table 1 indicates the concentration of the GFP plasmid in the suspension with respect to the cells in the suspension (calculated value).

Furthermore, "Cell processing amount" in Table 1 indicates the cell processing amount (Mcells) per minute (calculated value).

[Evaluation]

The gene introduction expression efficiency, the throughput, and the plasmid efficiency were evaluated as follows.

<Gene introduction expression efficiency>

The recovered suspension was diluted to 2 M ($\times 10^6$) cells/mL, and then 2 mL thereof were seeded in a well plate, accommodated in an incubator having a $CO_2$ concentration of 8% and an atmospheric temperature of 37°C, and statically cultured for 24 hours.

A cell concentration X after culturing for 24 hours was acquired using Vi-CELL XR (Beckman Coulter, Inc.), and a proportion Y of the gene-introduced cells was acquired using BD FACS Calibur (Becton, Dickinson and Company).

As a proportion of the gene-introduced cells obtained after culturing for 24 hours to the cells subjected to electroporation, the gene introduction expression efficiency (= Cell concentration X × Cell proportion Y ÷ 2 Mcells/mL × 100) (%) was calculated. The gene introduction expression efficiency was evaluated based on the following evaluation standard.

The evaluation standard was as follows. The results are shown in Table 1.

A: 50% or more
B: 35% or more and less than 50%
C: 20% or more and less than 35%
D: less than 20%

<Throughput>

The throughput was evaluated based on the cell processing amount per minute described above.

The evaluation standard was as follows. The results are shown in Table 1.

A: 400 Mcells/min or more
B: 100 Mcells/min or more and less than 400 Mcells/min
C: 10 Mcells/min or more and less than 100 Mcells/min
D: less than 10 Mcells/min

<Plasmid efficiency>

The plasmid efficiency was evaluated based on the plasmid concentration per cell described above.

The evaluation standard was as follows. The results are shown in Table 1.

A: less than 0.5 pg/cell
B: 0.5 pg/cell or more

[Table 1]

| | Example 1 | Example 2 | Example 3 | Example 4 | Example 5 | Example 6 | Example 7 | Comparative Example 1 | Comparative Example 2 | Comparative Example 3 | Comparative Example 4 |
|---|---|---|---|---|---|---|---|---|---|---|---|
| Thickness of suspension flow [mm] | 2 | 2 | 2 | 2 | 2 | 2 | 2 | 0.1 | 0.5 | 2 | 0.1 |
| Thickness of sheath flow (one side) [mm] | 2 | 0.5 | 2 | 0.5 | 0.5 | 0.5 | 0.5 | 0.1 | 0.1 | 0 | 0.1 |
| Thickness of recovery path (one side) [mm] | - | - | 1.5 | - | - | - | - | - | - | - | - |
| Width of flow channel [mm] | 2 | 2 | 2 | 2 | 2 | 2 | 2 | 0.2 | 2 | 2 | 0.2 |
| Cell concentration [Mcells/mL] | 40 | 40 | 40 | 120 | 140 | 140 | 140 | 40 | 40 | 120 | 120 |
| Cell volume fraction [%] | 17 | 17 | 17 | 50 | 59 | 59 | 59 | 17 | 17 | 50 | 50 |
| Plasmid concentration (in suspension) [ug/mL] | 30 | 30 | 30 | 30 | 30 | 30 | 30 | 30 | 30 | 30 | 30 |
| Plasmid concentration (in culture medium) [ug/mL] | 36.1 | 36.1 | 36.1 | 60.0 | 73.2 | 73.2 | 73.2 | 36.1 | 36.1 | 60.0 | 60.0 |
| Plasmid concentration per cell [pg/cell] | 0.75 | 0.75 | 0.75 | 0.25 | 0.21 | 0.21 | 0.21 | 0.75 | 0.75 | 0.25 | 0.25 |
| Pulse voltage [V] | 698 | 381 | 698 | 330 | 330 | 319 | 330 | 35 | 90 | 275 | 24.7 |

| | Example 1 | Example 2 | Example 3 | Example 4 | Example 5 | Example 6 | Example 7 | Comparative Example 1 | Comparative Example 2 | Comparative Example 3 | Comparative Example 4 |
|---|---|---|---|---|---|---|---|---|---|---|---|
| Electric field applied to suspension flow Em [V/cm] | 1376 | 1376 | 1376 | 1376 | 1423 | 1375 | 1423 | 1380 | 1377 | 1375 | 1376 |
| Pulse width [ms] | 3.5 | | 3.5 | 3.5 | | | 3.39 | 3.5 | 3.5 | 3.5 | 3.5 |
| Dilution rate | 3 | 3.5 1.5 | 1.5 | 1.5 | 3.5 1.5 | 3.5 1.5 | 1.5 | 3 | 1.4 | 1 | 3 |
| Feedback control | N | | N | N | | | | N | N | N | N |
| Cross-sectional area of cell flow [mm$^2$] | 4 | 4 | 4 | 4 | 4 | 4 | Y 4 | 0.02 | 1 | 4 480 | 0.02 |
| Flow rate of suspension [ml/min] | 4 | N 4 | 4 | 4 | N 4 | Y 4 | 4 | 0.02 | 1 | 4 | 0.02 |
| Flow rate of sheath (one side) [ml/min] | 4 | 1 | 4 | 1 | 1 | 1 | 1 | 0.2 | 0.2 | 0 | 0.2 |
| Cell processing amount [Mcells/min] | 160 | 160 | 160 | 480 | 560 | 560 | 560 | 0.8 | 40 | | 2.4 |
| Presence or absence of flow channel blockage | Not blocked | Not blocked | Not blocked | Not blocked | Not blocked | Not blocked | Not blocked | Not blocked | Not blocked | Not blocked | Blocked |
| Presence or absence of wall surface retention | Not retained | Not retained | Not retained | Not retained | Not retained | Not retained | Not retained | Not retained | Not retained | Retained | - |

(continued)

| | Example 1 | Example 2 | Example 3 | Example 4 | Example 5 | Example 6 | Example 7 | Comparative Example 1 | Comparative Example 2 | Comparative Example 3 | Comparative Example 4 |
|---|---|---|---|---|---|---|---|---|---|---|---|
| Gene introduction expression efficiency | C | B | B | A | B | A | A | C | D | D | - |
| Throughput | B | B | B | A | A | A | A | D | C | A | - |
| Plasmid efficiency | B | B | B | A | A | A | A | B | B | A | - |

As shown in Table 1, in the flow electroporation, according to the present invention in which the thickness of the suspension flow was set to 1 mm or more, suitable results were obtained for any of the gene introduction expression efficiency, the throughput, and the plasmid effect.

In addition, as shown in Examples 2 and 3, the gene introduction expression efficiency could be further improved by setting the dilution rate of the suspension to 2 times or less. In addition, as shown in Example 4, the gene introduction expression efficiency, the throughput, and the plasmid effect could be improved by setting the cell volume fraction in the suspension to 20% or more.

Furthermore, as shown in Examples 6 and 7, the gene introduction expression efficiency could be improved by performing the feedback control using the conductivity of the suspension.

On the other hand, in Comparative Examples 1 and 2 in which the thickness of the suspension flow was less than 1 mm, the throughput was low; and particularly, in Comparative Example 2 in which the cell flow rate was increased, the gene introduction expression efficiency was also low.

In addition, in Comparative Example 3 in which the sheath flow was not used, it was confirmed that the cell flow retained near the wall surface of the flow channel, and the gene introduction expression efficiency was low.

Furthermore, in Comparative Example 4 in which the thickness of the suspension flow was less than 1 mm and the cell volume fraction was set to 50%, the flow channel was closed even though the sheath flow was present.

Flow electroporation was performed in the same manner as in Example 4 and Example 6, except that the thickness of the suspension flow was set to 1 mm, 3 mm, 5 mm, or 8 mm. Even in this case, sufficient gene introduction expression efficiency and throughput were obtained.

Flow electroporation was performed in the same manner as in Example 4 and Example 6, except that the thickness (one side) of the sheath flow was set to 0.1 mm or 1 mm. Even in this case, sufficient gene introduction expression efficiency and throughput were obtained.

Flow electroporation was performed in the same manner as in Example 4 and Example 6, except that the cell concentration was set to 50 Mcells/mL or 80 Mcells/mL. Even in this case, sufficient gene introduction expression efficiency and throughput were obtained.

Flow electroporation was performed in the same manner as in Example 4 and Example 6, except that the flow channel device as shown in FIG. 7 was used. Even in this case, sufficient gene introduction expression efficiency and throughput were obtained.

Flow electroporation was performed in the same manner as in Example 4 and Example 6, except that the plasmid DNA to be introduced was set to three types of plasmid DNA for adeno-associated virus (AAV) production. Even in this case, sufficient gene introduction expression efficiency and throughput were obtained. In addition, favorable AAV production was obtained.

From the above results, the effect of the present invention is clear.

The present invention can be suitably used for manufacturing a gene therapy drug or the like.

Explanation of References

10, 50: flow channel device
12, 52, 100: first substrate
14, 54, 102: second substrate
16, 64: flow channel substrate
16a: first flow channel plate
16b: second flow channel plate
16c: third flow channel plate
16d: fourth flow channel plate
16e: fifth flow channel plate
18, 20, 60, 62, 106, 108: electrode
24, 70: suspension inflow path
26a, 26b, 76a, 76b: sheath liquid inflow path
28a, 28b: sheath liquid flow channel
30, 74: main flow channel
30a: suspension flow channel
32, 72: discharge port
52a, 54a, 56a, 58a: through-hole
52b, 54b: recovery path
64a, 64b: protrusion

80: suspension container
82: liquid feeding pump
84: measurement jig
86: conductivity calculation unit
90: operation and power supply control device
92: pulse power supply
104: flow channel
m: suspension
s: sheath liquid

## Claims

1. An electroporation method in which an electric field is applied to a suspension containing a bioactive substance and a biologically derived substance by an electrode pair to introduce the bioactive substance into the biologically derived substance, the electroporation method comprising:

   forming sheath flows of a sheath liquid which flows together with the suspension and comes into contact with electrodes constituting the electrode pair, the sheath liquid being a liquid other than the suspension; and
   providing a restriction region through which only a suspension flow consisting of the suspension flows, in which a thickness of the suspension flow flowing through the restriction region in a separation direction of the electrodes constituting the electrode pair is 1 to 10 mm, and combining the suspension flow and the sheath flows downstream of the restriction region.

2. The electroporation method according to claim 1,
   wherein a total thickness of the sheath flows in the separation direction of the electrodes in a case of combining with the suspension flow is equal to or less than the thickness of the suspension flow in the separation direction of the electrodes in the restriction region.

3. The electroporation method according to claim 1,
   wherein, in a case where the sheath liquid is recovered downstream of the electrode pair, a dilution rate of the suspension in a mixed solution of the suspension and the sheath liquid after the sheath liquid is recovered is 2 times or less.

4. The electroporation method according to any one of claims 1 to 3,
   wherein a volume fraction of the biologically derived substance in the suspension is 10% or more.

5. The electroporation method according to claim 4,
   wherein the volume fraction of the biologically derived substance in the suspension is 40% or more.

6. The electroporation method according to any one of claims 1 to 5,
   wherein the suspension is a cell suspension, and the sheath liquid is a culture medium used for culturing cells of the cell suspension.

7. The electroporation method according to any one of claims 1 to 6,
   wherein, before the electric field is applied, a conductivity of the suspension or a concentration of the biologically derived substance in the suspension is measured, and the electric field or a pulse width applied to the electrode pair is adjusted according to a measurement result.

8. The electroporation method according to any one of claims 1 to 7,
   wherein a flow rate of the suspension is 1 mL/min or more.

9. The electroporation method according to any one of claims 1 to 8,
   wherein a flow rate of the sheath liquid is 0.1 mL/min or more.

10. An electroporation method in which an electric field is applied to a suspension containing a bioactive substance and a biologically derived substance by an electrode pair to introduce the bioactive substance into the biologically derived substance, the electroporation method comprising:

forming sheath flows of a sheath liquid which flows together with the suspension and comes into contact with electrodes constituting the electrode pair, the sheath liquid being a liquid other than the suspension; and setting a thickness of a suspension flow consisting of the suspension in a separation direction of the electrodes to 1 to 10 mm in at least a part in the electrode pair.

11. A method for manufacturing a useful substance, comprising:
the electroporation method according to any one of claims 1 to 10.

12. A flow channel device used for electroporation in which an electric field is applied to a suspension containing a bioactive substance and a biologically derived substance by an electrode pair to introduce the bioactive substance into the biologically derived substance, the flow channel device comprising:

a main flow channel;
an electrode pair which applies an electric field to a liquid flowing through the main flow channel;
a supply port which supplies the suspension to the main flow channel;
a suspension flow channel which has a restriction region through which only the suspension flows; and
a sheath liquid flow channel through which only a sheath liquid which is a liquid other than the suspension flows,
wherein the sheath liquid flow channel is disposed such that the sheath liquid flows downstream of the supply port and upstream of the electrode pair, and
a thickness of the restriction region in a separation direction of electrodes constituting the electrode pair is 1 to 10 mm.

13. The flow channel device according to claim 12,
wherein an angle formed by the main flow channel and the sheath liquid flow channel is 60° or less.

14. The flow channel device according to claim 12 or 13,
wherein the sheath liquid flow channel has a region in which the flow channel is widened in a width direction toward a downstream side.

15. The flow channel device according to any one of claims 12 to 14,
wherein a width of the main flow channel is 2 mm or more.

16. The flow channel device according to any one of claims 12 to 15, further comprising, downstream of the electrodes:
a discharge port for discharging the sheath flows from the main flow channel.

17. An electroporation apparatus comprising:

the flow channel device according to any one of claims 12 to 16; and
a pair of contactors for energizing the electrodes of the flow channel device.

# FIG. 1

# FIG. 2

# FIG. 3

## FIG. 4

# FIG. 5

## FIG. 6

## FIG. 7

## FIG. 8

## FIG. 9

## FIG. 10

## FIG. 11

## INTERNATIONAL SEARCH REPORT

| International application No. |
|---|
| **PCT/JP2024/020041** |

**A. CLASSIFICATION OF SUBJECT MATTER**

*C12N 13/00*(2006.01)i; *C12M 1/00*(2006.01)i; *C12M 1/42*(2006.01)i; *C12N 15/09*(2006.01)i; *C12N 15/87*(2006.01)i; *C12P 1/00*(2006.01)i

FI:   C12N13/00; C12M1/42; C12M1/00 A; C12P1/00 Z; C12N15/09 Z; C12N15/87 Z

According to International Patent Classification (IPC) or to both national classification and IPC

**B. FIELDS SEARCHED**

Minimum documentation searched (classification system followed by classification symbols)

C12N13/00; C12M1/00; C12M1/42; C12N15/09; C12N15/87; C12P1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2023-503934 A (THE CHARLES STARK DRAPER LABORATORY, INC.) 01 February 2023 (2023-02-01)<br>claims 1-20, paragraphs [0027]-[0030], [0051], [0058], [0070], fig. 4 | 1-17 |
| A | US 2019/0338235 A1 (THE CHARLES STARK DRAPER LABORATORY, INC.) 07 November 2019 (2019-11-07)<br>fig. 1 | 1-17 |
| A | US 2020/0399578 A1 (CYTEQUEST, INC.) 24 December 2020 (2020-12-24)<br>fig. 5 | 1-17 |
| A | US 2022/0162540 A1 (CELLIX LIMITED) 26 May 2022 (2022-05-26)<br>paragraphs [0138]-[0139], fig. 2 | 1-17 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 August 2024** | **20 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/020041**

| Patent document cited in search report | | | Publication date (day/month/year) | Patent family member(s) | | | Publication date (day/month/year) |
|---|---|---|---|---|---|---|---|
| JP | 2023-503934 | A | 01 February 2023 | US | 2021/0155889 | A1 | |
| | | | | claims 1-20, paragraphs [0038]-[0041], [0062], [0069], [0081], fig. 4 | | | |
| | | | | WO | 2021/102304 | A1 | |
| | | | | CN | 115413295 | A | |
| US | 2019/0338235 | A1 | 07 November 2019 | EP | 3564355 | A1 | |
| | | | | fig. 1 | | | |
| US | 2020/0399578 | A1 | 24 December 2020 | WO | 2020/232437 | A1 | |
| | | | | fig. 5 | | | |
| | | | | EP | 3969580 | A1 | |
| | | | | CN | 114127280 | A | |
| US | 2022/0162540 | A1 | 26 May 2022 | WO | 2020/201206 | A1 | |
| | | | | pp. 26-29, fig. 2 | | | |
| | | | | EP | 3946733 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007007430 A
- US 11225638 B

- WO 2015005430 A